# EUROPEAN PATENT APPLICATION

(11) **EP 2 929 838 A1**
(43) Date of publication of application: **14.10.2015**
(21) Application number: 13861125.6
(22) Date of filing: 21.11.2013
(51) Int. Cl.: A61B 5/1455

(54) **PULSE OXIMETRY SYSTEM, AND SUBSYSTEM AND COMMUNICATION CONVERSION DEVICE FOR CONSTRUCTING SAID PULSE OXIMETRY SYSTEM**

(30) Priority: 06.12.2012 JP 2012266974
(71) Applicant: Konica Minolta, Inc., Tokyo 100-7015 (JP)
(72) Inventor: TATEDA, Norihiro, Tokyo 100-7015 (JP); KANAZAWA, Masaharu, Tokyo 100-7015 (JP); KAMEZAWA, Hitoshi, Tokyo 100-7015 (JP)
(74) Representative: Hoffmann Eitle
(86) International application number: PCT/JP2013/081339
(87) International publication number: WO 2014/087844

(57) **Abstract**

A pulse oximetry system includes: a pulse oximeter that creates biological information data; a communication conversion device that receives the biological information data from the pulse oximeter and converts the communication system of the biological data into a communication system fitted to a display device; and the display device that receives the biological information data from the communication conversion device. A storage unit of the communication conversion device stores a plurality of candidate communication systems. A user can select a communication system fitted to the display device from the candidate communication systems, and set the selected communication system as a communication system after conversion. Accordingly, in case of presence of a communication system fitted to the communication system of the display device in the plurality of candidate communication systems, communication is allowed via the communication conversion device even when the communication systems of the display device and the pulse oximeter are different.

## Description

### Technical Field

The present invention relates to a pulse oximetry system, and a subsystem and a communication conversion device for constructing this pulse oximetry system.

### Background Art

A pulse oximeter capable of measuring oxygen saturation (SpO2) in blood is known. According to this type of pulse oximeter, light is applied to a living site of a testee from a measuring portion of the pulse oximeter attached to the living site to calculate SpO2 based on the quantity of light transmitted through the living site or reflected on the living site (for example, see Patent Literature 1).

In constructing a pulse oximeter, in general, a device which has both the foregoing measuring function and a transmitting function for transmitting measurement data converted into electric signals or the like is disposed on the testee side, while a device (such as a display device) which has both a receiving function for receiving measurement data and a function for performing predetermined data processing for the measurement data is disposed at a position away from the testee and the device on the testee side. This arrangement division of the constituent elements associated with calculation of SpO2 into the measuring device and the data processing device decreases the size of the measuring device (device disposed on the testee side), and thus reduces a burden imposed on the testee during attachment of the device.

In addition, an invention of wireless telemetry systems for medical use is disclosed in Patent Literature 2, as a technology which divides medical devices into a testee side device and a receiver side device.

### Citation List

### Patent Literatures

Patent Literature 1: JP 2005-110816 A
Patent Literature 2: Patent No. 4,055,102

### Summary of Invention

### Technical Problem

According to configurations of the systems disclosed in Patent Literatures 1 and 2, the communication system between the device disposed on the testee side and the device disposed on the receiver side is regulated by a corresponding single communication profile, wherefore communication of the measurement data between both the devices is achievable in a normal condition.

On the other hand, when the respective devices have different communication profiles, such as a case when the device disposed on the testee side and the device disposed on the receiver side are produced by different manufacturers, such a problem may arise that communication between the respective devices in the normal condition becomes difficult.

The present invention has been developed to solve the aforementioned problems. Provided according to the present invention are a pulse oximetry system, and a subsystem and a communication conversion device for constructing this pulse oximetry system, which are capable of realizing communication between a pulse oximeter creating biological information data and a data processing device receiving transmission of the biological information data from the pulse oximeter even when communication profiles of these devices are different.

### Solution to Problem

In order to achieve at least one of the above objects, a pulse oximetry system reflecting an aspect of the present invention includes: (a) a pulse oximeter attached to a finger of a test living body to create biological information data containing information for specifying oxygen saturation in blood of the test living body, and transmit the biological information data in a predetermined first communication system; (b) a data processing device that performs predetermined data processing for the biological information data; and (c) a communication conversion device that includes a storage unit storing each communication profile of a plurality of candidate communication systems, and a setting unit selecting a second communication system fitted to communication with the data processing device from the plurality of candidate communication systems, and setting a communication profile of the second communication system, wherein the communication conversion device transmits the biological information data to the data processing device in the second communication system after receiving the biological information data in the first communication system from the pulse oximeter.

In order to achieve at least one of the above objects, a subsystem for constructing a pulse oximetry system reflecting an aspect of the present invention includes (a) a pulse oximeter attached to a finger of a test living body to create biological information data containing information for specifying oxygen saturation in blood of the test living body, and transmit the biological information data in a predetermined first communication system, and (b) a communication conversion device that includes a storage unit storing each communication profile of a plurality of candidate communication systems, and a setting unit selecting a second communication system fitted to communication with a data processing device corresponding to a data transmission destination from the plurality of candidate communication systems, and setting a communication profile of the second communication system, wherein the communication conversion device transmits the biological information data to the data processing device in the second communication system after receiving the biological information data in the first communication system from the pulse oximeter.

In order to achieve at least one of the above objects, a communication conversion device used in combination with a pulse oximeter attached to a finger of a test living body to create biological information data containing information for specifying oxygen saturation in blood of the test living body, and transmit the biological information data in a predetermined first communication system, the communication conversion device reflecting an aspect of the present invention includes: a storage unit storing each communication profile of a plurality of candidate communication systems; a setting unit selecting a second communication system fitted to communication with a data processing device corresponding to a transmission destination of the biological information data from the plurality of candidate communication systems, and setting a communication profile of the second communication system; and transmitting means that transmits the biological information data to the data processing device in the second communication system after receiving the biological information data in the first communication system from the pulse oximeter.

### Advantageous Effects of Invention

A communication conversion device included in a pulse oximetry system according to the present invention includes a storage unit storing each communication profile of a plurality of candidate communication systems, and a setting unit selecting a second communication system fitted to communication with a data processing device from the plurality of candidate communication systems, and setting a communication profile of the second communication system. The communication conversion device has a function of transmitting the biological information data to the data processing device in the second communication system after receiving the biological information data in a first communication system from a pulse oximeter.

In this case, the communication profile is converted by the communication conversion device provided in the course of communication between the pulse oximeter and the data processing device even when the communication profile of the pulse oximeter creating biological information data is different from the communication profile of the data processing device receiving the biological information data and performing predetermined data processing for the biological information data. Accordingly, communication of biological information data is achievable between these devices.

### Brief Description of Drawings

Fig. 1 is a view schematically illustrating a pulse oximetry system according to an embodiment.
Fig. 2 is a view schematically illustrating an external appearance of a pulse oximeter according to the embodiment.
Fig. 3 is a view schematically illustrating the external appearance of the pulse oximeter according to the embodiment.
Fig. 4 is a view schematically illustrating the external appearance of the pulse oximeter according to the embodiment.
Fig. 5 is a view illustrating an X-Z cross section taken along a chain line III-III in Fig. 3.
Fig. 6 is a view illustrating a Y-Z cross section taken along a chain line V-V in Fig. 5.
Fig. 7 is a block diagram illustrating a functional configuration of the pulse oximeter according to the embodiment.
Fig. 8 is a block diagram illustrating a functional configuration of the pulse oximeter according to the embodiment.
Fig. 9 illustrates a state of insertion of the pulse oximeter into a finger according to the embodiment.
Fig. 10 illustrates a state of insertion of the pulse oximeter into a finger according to the embodiment.
Fig. 11 illustrates a state of insertion of the pulse oximeter into a finger according to the embodiment.
Fig. 12 is a view schematically illustrating an external appearance of a communication conversion device according to the embodiment.
Fig. 13 is a view illustrating an X-Z cross section taken along a dotted line XII-XII in Fig. 12.
Fig. 14 is a view schematically illustrating the pulse oximetry system when a plurality of display devices are present according to the embodiment.
Fig. 15 is a block diagram illustrating a functional configuration of the communication conversion device according to the embodiment.
Fig. 16 is a block diagram illustrating a functional configuration of the communication conversion device according to the embodiment.
Fig. 17 is a block diagram illustrating a functional configuration of the display device according to the embodiment.
Fig. 18 is a block diagram illustrating a functional configuration of the display device according to the embodiment.
Fig. 19 is a block diagram illustrating a functional configuration of the pulse oximetry system according to the embodiment.
Fig. 20 is a block diagram illustrating a functional configuration of a pulse oximetry system according to a modified example.
Fig. 21 is a block diagram illustrating a functional configuration of a pulse oximetry system according to a modified example.

### Description of Embodiments

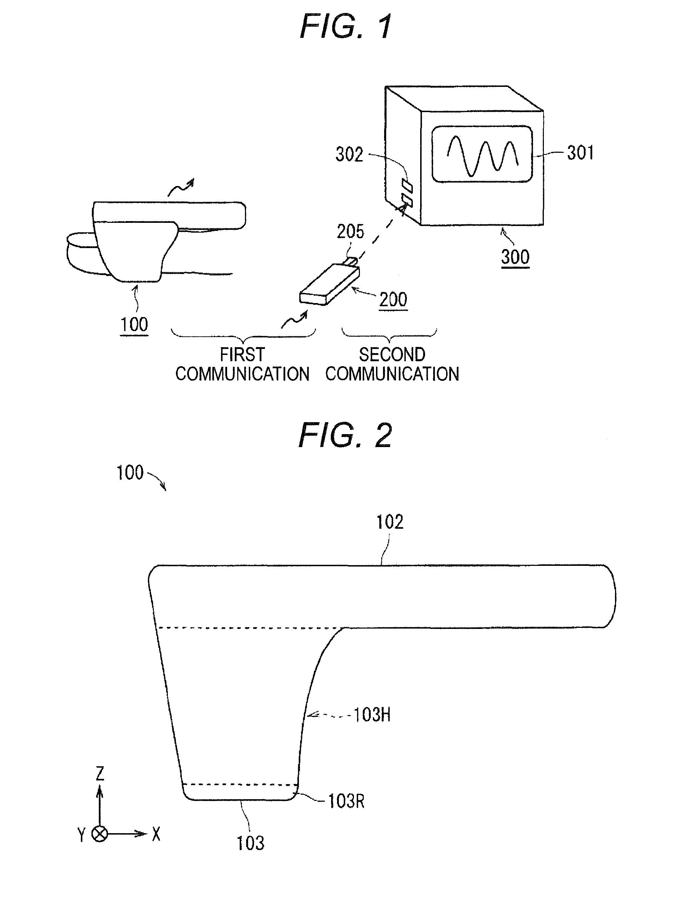

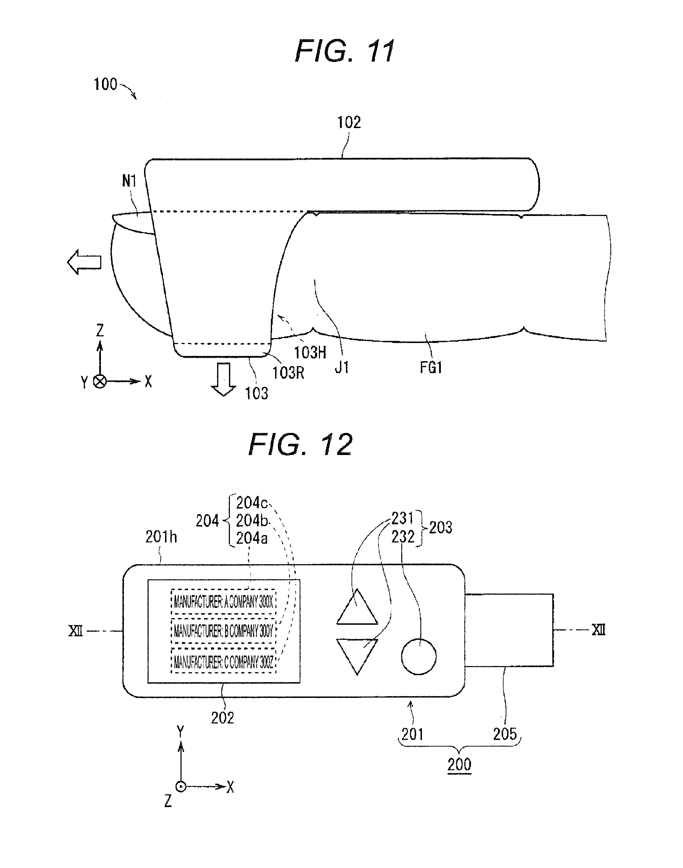

An embodiment according to the present invention is hereinafter described with reference to the drawings. In the respective figures, parts having similar configurations and functions have been given similar reference numbers, and the same explanation is not repeated in the following description. The respective figures are only schematic illustrations, and do not show accurate sizes, positional relationships and the like of the respective structures. Each of Figs. 2 to 6, and Figs. 9 to 13 contains a right-handed X-Y-Z coordinate system which defines a direction along the longitudinal direction of a pulse oximeter 100 and a communication conversion device 200 (rightward direction as viewed in Figs. 2 and 12) as the +X direction.

### <(1) Configuration and Operation of Embodiment>

### <(1-1) Pulse Oximetry System>

Fig. 1 is a conceptual view illustrating a configuration of the pulse oximetry system 1 according to the embodiment of the present invention.

As illustrated in Fig. 1, the pulse oximetry system 1 according to this embodiment generally includes the pulse oximeter 100 which creates biological information data, the communication conversion device 200 which receives the biological information data from the pulse oximeter 100 and converts the communication system of the biological information data into a communication system fitted to a display device 3, and the display device 300 which receives the biological information data from the communication conversion device 200 and displays medical information on a display unit 301 based on the biological information data.

The pulse oximeter 100 is a measuring device attached to a test living body (such as a testee) to create biological information data containing information for specifying oxygen saturation in blood of the testee. The created biological information data is transmitted from the pulse oximeter 100 to the communication conversion device 200 in a communication system fitted to communication with the pulse oximeter 100 (hereinafter referred to as "first communication system"). According to this embodiment, the first communication system is fitted to a wireless system (see Fig. 1).

The communication conversion device 200 receives the biological information data from the pulse oximeter 100 in the first communication system. The communication conversion device 200 converts the communication profile of the first communication system of the received biological information data into a communication profile of a communication system fitted to communication with the display device 3 (hereinafter referred to as "second communication system"), and transmits the biological information data in the converted communication profile. In other words, the communication conversion device 200 is capable of handling different communication profiles for each of the receiving side and the transmitting side, and therefore performs a function of relaying devices having different communication profiles.

The communication conversion device 200 includes a connector 205 disposed on an exterior part and functioning as a physical interface (hereinafter abbreviated as "I/F") in the form of a USB (Universal Serial Bus). On the other hand, the display device 300 includes a general-purpose port 302 disposed on an exterior part and functioning as a physical I/F corresponding to the connector 205. In this configuration, the communication conversion device 200 and the display device 300 are electrically connected with each other by detachable insertion of the connector 205 into the port 302. Accordingly, the second communication system in this embodiment is fitted to serial communication achieved by the connector 205 and the port 302 (see Fig. 1).

According to the pulse oximetry system 1 in this embodiment, therefore, communication between the pulse oximeter 100 and the display device 300 (data processing device) via the communication conversion device 200 is realized in a state of electric connection between the connector 205 provided on an external part of the communication conversion device 200 and the general-purpose port 302 provided on an external part of the display device 300 (data processing device). Accordingly, the communication conversion device 200 is applicable to use in combination with various types of data processing devices equipped with the genera-purpose port 302.

The display device 300 receives biological information data from the communication conversion device 200 in the second communication system, and displays medical information on the display unit 301 based on the biological information data.

In the following description, communication between the pulse oximeter 100 and the communication conversion device 200 is referred to as "first communication", while communication between the communication conversion device 200 and the display device 300 is referred to as "second communication".

According to the pulse oximetry system 1 in this embodiment, therefore, communication from the pulse oximeter 100 to the display device 300 passes through the communication conversion device 200 capable of converting a communication profile. Accordingly, the biological information data transmitted from the pulse oximeter 100 is receivable by the display device 300 even when communication profiles of the first communication and the second communication are not directly fitted to each other. In this specification, the term "communication system" refers to a general system specifying communication between devices (such as physical I/F and communication profile), and therefore expresses a wider conceptual range than the term "communication profile".

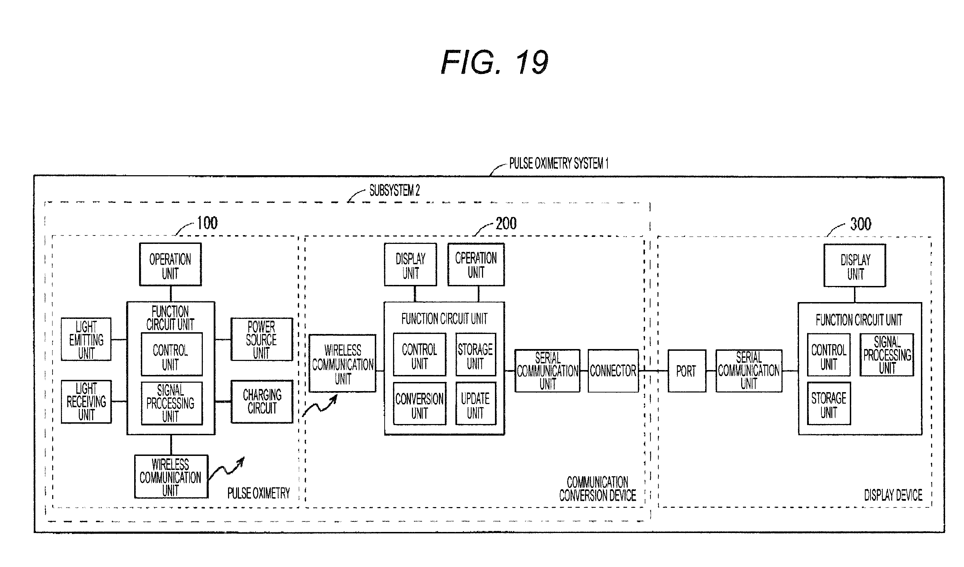

The respective devices constituting the pulse oximetry system 1 (pulse oximeter 100, communication conversion device 200, and display device 300) are hereinafter described in detail. A general functional block diagram of the pulse oximetry system 1 is illustrated in Fig. 19.

### <(1-2) Pulse Oximeter 100>

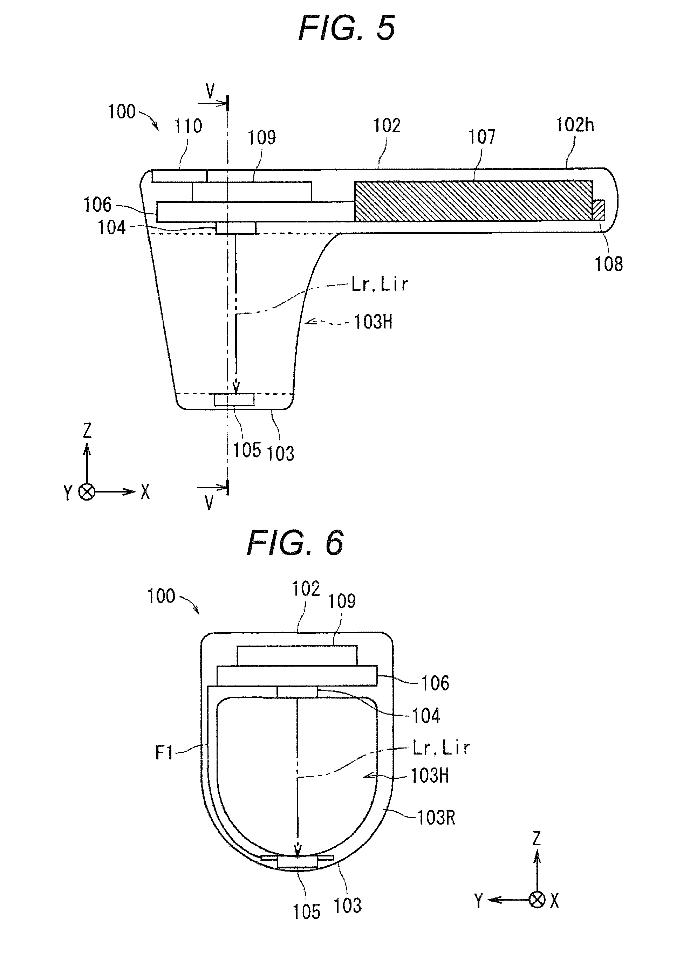

The pulse oximeter 100 according to this embodiment is a portable device which obtains a digital value (SpO2 value) associated with oxygen saturation in blood, based on a signal output from a light receiving unit 105 of the pulse oximeter 100 when the light receiving unit 105 receives light emitted from a light emitting unit 104 of the pulse oximeter 100 and transmitted through a finger (see Figs. 5 and 6).

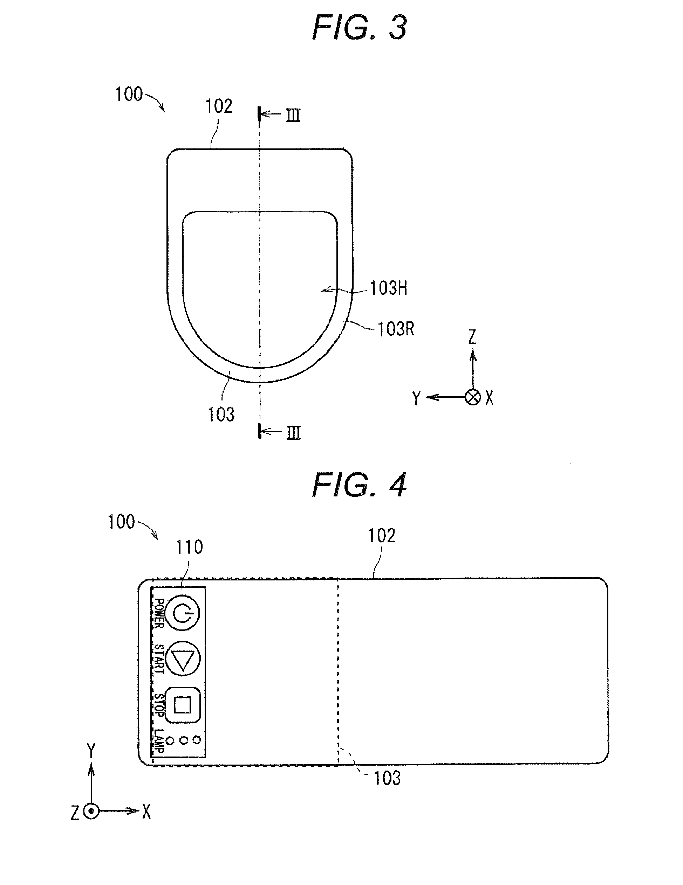

Figs. 2 to 4 are views schematically illustrating an external appearance of the pulse oximeter 100. Figs. 2, 3, and 4 are a side view, a front view, and a top view illustrating the external appearance of the pulse oximeter 100, respectively. Figs. 5 and 6 are views schematically illustrating a configuration of the pulse oximeter 100. Fig. 5 illustrates an X-Z cross section taken along a chain line III-III in Fig. 3. Fig. 6 illustrates a Y-Z cross section taken along a chain line V-V in Fig. 5.

As illustrated in Figs. 2 to 4, the pulse oximeter 100 includes a body 102 and an attachment portion 103.

The body 102 includes a housing 102h, and further includes a function circuit unit 106, a power source unit 107, a charging circuit 108, a wireless communication unit 109, and an operation unit 110, each of which units 106 to 110 is housed in the housing 102h. The housing 102h has a substantially rectangular solid shape, for example.

The attachment portion 103 is fixed to the body 102, and functions as a portion for holding a finger of a living body at the time of measurement of various information about the living body. The attachment portion 103 may be made of any material as long as the material contains an elastic body generating an elastic force for holding the finger, for example. The elastic body is made of high polymer material such as rubber, or constituted by a spring, for example. More specifically, the attachment portion 103 in a mode may include resin such as rubber having elasticity and constituting substantially the whole of the attachment portion 103, or resin containing a substantially U-shaped flat spring embedded in the resin, for example.

As illustrated in Figs. 5 and 6, the pulse oximeter 100 includes the light emitting unit 104 and the light receiving unit 105. The light emitting unit 104 and the light receiving unit 105 are so positioned as to face to each other, between which units 104 and 105 there lies an area where the finger is placed and held by the attachment portion 103. Positioning of the light emitting unit 104 and the light receiving unit 105 on either the body 102 side or the attachment portion 103 side may be arbitrarily determined.

When the attachment portion 103 includes an annular portion 103R which contains an insertion hole 103H, for example, the insertion hole 103H receives the living body finger inserted in the -X direction. In this case, the insertion hole 103H functions as the area where the finger is held by the attachment portion 103. This structure allows extremely easy attachment of the pulse oximeter 100 to the finger only by insertion of the finger into the insertion hole 103H. When such a configuration is adopted which allows deformation of the annular portion 103R in a closing direction of the insertion hole 103H by an elastic force generated by the elastic body of the attachment portion 103, the pulse oximeter 100 is attachable to the finger in a stable condition.

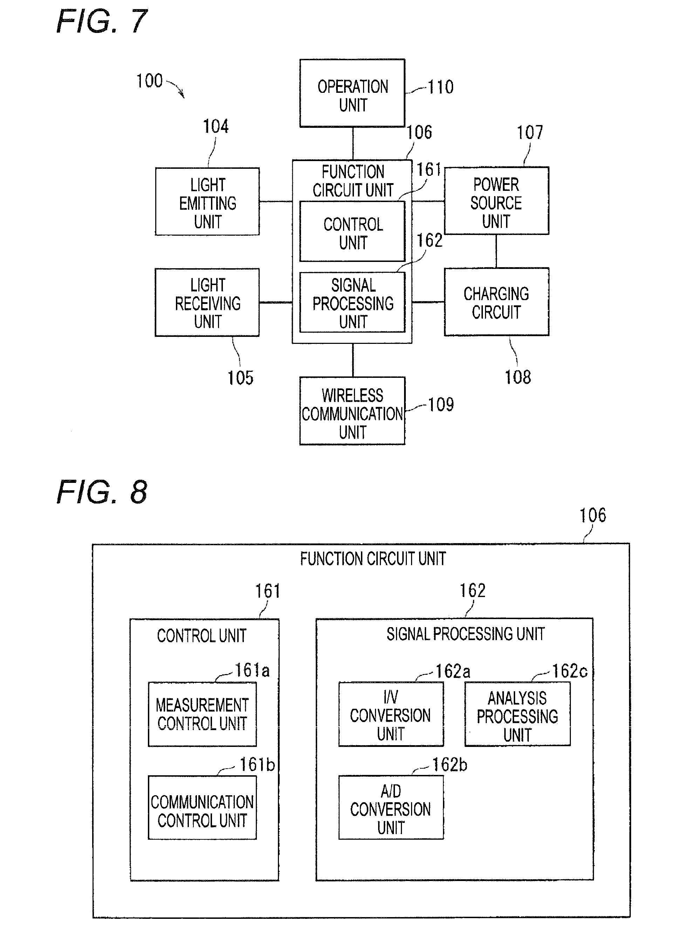

Figs. 7 and 8 are block diagrams illustrating a functional configuration of the pulse oximeter 100.

As illustrated in Fig. 7, the pulse oximeter 100 includes the light emitting unit 104, the light receiving unit 105, the function circuit unit 106, the power source unit 107, the charging circuit 108, the wireless communication unit 109, and the operation unit 110.

The light emitting unit 104 emits light toward the light receiving unit 105 when receiving supply of power from the power source unit 107 under the control by the functional circuit unit 106. Fig. 5 indicates a traveling route (optical path) of this light in a two-dot chain line. The light emitting unit 104 includes a portion for emitting light having a wavelength λ1 in a red area, and a portion for emitting light having a wavelength λ2 as infrared light. The light emitting unit 104 having this structure may be constituted by an LED (Light Emitting Diode), for example. At the time of measurement, repetitive pulsed light emission is conducted so that red light Lr of the wavelength λ1, and infrared light Lir of the wavelength λ2 can be emitted alternately in time from the light emitting unit 104.

The light receiving unit 105 outputs a current signal at a level corresponding to the intensity of the received light to a signal processing unit 162 described later. For example, the light receiving unit 105 includes a photoelectric conversion element such as a silicon photo diode sensitive to at least light of the wavelength λ1 and light of the wavelength λ2. The light receiving unit 105 receives light contained in the lights of the wavelengths λ1 and λ2 from the light emitting unit 104 and transmitted through biological tissues of the finger in a state of insertion of the finger through the insertion hole 103H. The light receiving unit 105 is electrically connected with the function circuit unit 106 via wiring. For example, the light receiving unit 105 in a mode may be disposed on flexible printed circuits (FPC) F1 electrically connected with the function circuit unit 106. The current signal output from the light receiving unit 105 is transmitted to the function circuit unit 106 through this connection.

At the time of measurement, the red light Lr of the wavelength λ1 and the infrared light Lir of the wavelength λ2 are emitted alternately in time from the light emitting unit 104, and received by the light receiving unit 105 as a light receiving action synchronized with a light emitting action of the light emitting unit 104. The light emitting action of the light emitting unit 104 and the light receiving action of the light receiving unit 105 are controllable by a control unit 161 described later. The actions for transmitting and receiving the respective lights Lr and Lir are repeated in a cycle approximately in a range from 1/100 (sec.) to 1/30 (sec) (inclusive), for example.

When the positions of the light emitting unit 104 and the light receiving unit 105 are determined such that the light emitting unit 104 is disposed on the body 102 side, and that the light receiving unit 105 is disposed on the attachment portion 103 side, the wiring route for supplying power to the light emitting unit 104 may be reduced. This arrangement therefore may decrease noise effect on the function circuit unit 106 and the like produced by power supply to the light emitting unit 104.

The function circuit unit 106 includes the control unit 161 and the signal processing unit 162. The function circuit unit 106 may be any types constituted by various electronic parts, integrated circuit parts, a CPU and others. As illustrated in Fig. 8, the control unit 161 includes a measurement control unit 161a, a communication control unit 161b, and a charging circuit control unit (not shown). The signal processing unit 162 includes a current-voltage conversion unit (hereinafter referred to as I/V conversion unit) 162a, an analog digital conversion unit (hereinafter referred to as A/D conversion unit) 162b, and an analysis processing unit 162c.

The measurement control unit 161a controls operations of the light emitting unit 104 and the light receiving unit 105. In this embodiment, the red light Lr of the wavelength λ1 and the red light Lir of the wavelength λ2 are emitted alternately in time from the light emitting unit 104 in a cycle of 1/100 (sec.) for each, for example. The communication control unit 161b controls data communication of the wireless communication unit 109 described later.

The I/V conversion unit 162a converts the current signal cyclically output from the light receiving unit 105 into a voltage signal. This voltage signal is a signal associated with analog pulse waves (referred to as pulse signal as well). The A/D conversion unit 162b converts the analog pulse signal output from the I/V conversion unit 162a into a digital pulse signal to produce a digital value associated with pulse waves.

The analysis processing unit 162c carries out predetermined data analysis based on the digital pulse signal output from the A/D conversion unit 162b. This analysis calculates various types of values such as light amounts and pulse wave amplitudes of the respective lights Lr and Lir received by the light receiving unit 105, a ratio of the amplitude of the red light Lr and the amplitude of the infrared light Lir, a value of oxygen saturation (SpO2 value) in blood, a pulse count, a pulse wave interval (cycle), and others. According to the present invention, the term "biological information data" refers to data containing information obtained based on measurement by using the pulse oximeter 100 to specify oxygen saturation in blood, and thus refers to data not dependent on data format. Accordingly, the foregoing analog pulse signal and digital pulse signal both correspond to "biological information data".

Each of the measurement control unit 161a, the communication control unit 161b, and the analysis processing unit 162c may be constituted by a dedicated electronic circuit, or may be realized by a program executed by a microprocessor or a DSP (Digital Signal Processor).

The power source unit 107 includes a secondary battery such as a nickel-hydrogen storage cell and a lithium ion battery, for example. Power is supplied from the power source unit 107 to the respective constituent elements of the pulse oximeter 100 such as the function circuit unit 106 and the light emitting unit 104. This structure eliminates the necessity of a mechanism provided on the body 102 for replacement of a primary battery such as a dry cell. This structure therefore realizes a simplified and irrefrangible configuration of the body 102.

The charging circuit 108 is a circuit for charging the second battery of the power source unit 107. In a possible mode, the secondary battery is charged by connection between a battery charger and a terminal electrically connected with the secondary battery, for example. This structure simplifies the structure for charging the secondary battery. When the charging circuit 108 performs contactless charging of the secondary battery, i.e., when the charging circuit 108 has a circuit realizing contactless charging of the secondary battery, the terminal or the like connecting with the battery charger or the like is unnecessary. This structure further simplifies the structure for charging the secondary battery. The contactless charging method adoptable herein may be a method utilizing electromagnetic induction of a coil, for example. This structure may eliminate the necessity of providing a mechanism (such as openable and closable cover) on the body 2 for replacement of a dry cell or other types of primary battery. Accordingly, this structure may realize a simplified and irrefrangible configuration of the body 2.

The wireless communication unit 109 transmits data obtained by the signal processing unit 162 to the communication conversion device 200 in the first communication system. The first communication system may be Bluetooth (registered trademark) standards (IEEE 802.15.1). The transmitted data is processed and displayed on the display device 300 or the like, wherefore the pulse oximeter 100 is not required to contain a structure for analysis and storage of signals, and a display unit for displaying measurement results. Accordingly, reduction of the size, power consumption, and manufacturing cost of the pulse oximeter 100 is achievable. Particularly, decrease in the size of the pulse oximeter 100 reduces a burden imposed on the testee at the time of attachment (minimal invasion). As noted above, the first communication system is fitted to wireless communication.

It is assumed herein that the signal processing unit 162 obtains a digital value associated with at least any one of the oxygen saturation value (SpO2 value) in blood, the pulse count, and the pulse wave interval (cycle) based on the digital pulse signal. In this case, data on the digital value associated with at least any one of the oxygen saturation value (SpO2 value) in blood, the pulse count, and the pulse wave interval (cycle) obtained by the signal processing unit 162 is transmittable from the wireless communication unit 109 to the communication conversion device 200. Accordingly, the communication conversion device 200 can easily obtain useful information without the necessity of particular calculations or the like by the communication conversion device 200 after receiving the data from the wireless communication unit 109.

The function circuit unit 106 may include various types of memories for storing data obtained by the signal processing unit 162. These memories store measurement data obtained by measurement by the use of the biological information measuring device 1 attached to an outdoor emergency patient for whom medical equipment such as a monitoring device and a treatment device is difficult to be prepared. After this patient is conveyed to a hospital or an ambulance, the measurement data is read from the memories and transmitted to a monitoring device or a treatment device to acquire the status of the patient immediately after the onset of the sudden illness in a retrospective manner for time-lag use of the biological information measuring device 1.

The operation unit 110 includes a power button, a measurement start button, and a measurement stop button, for example. The power button is a button for switching supply and non-supply of power from the power source unit 107 to the respective units of the pulse oximeter 100. The measurement start button is a button for starting measurement of the oxygen saturation value (SpO2 value) in blood, for example. The measurement stop button is a button for ending measurement of the oxygen saturation value (SpO2 value) in blood, for example.

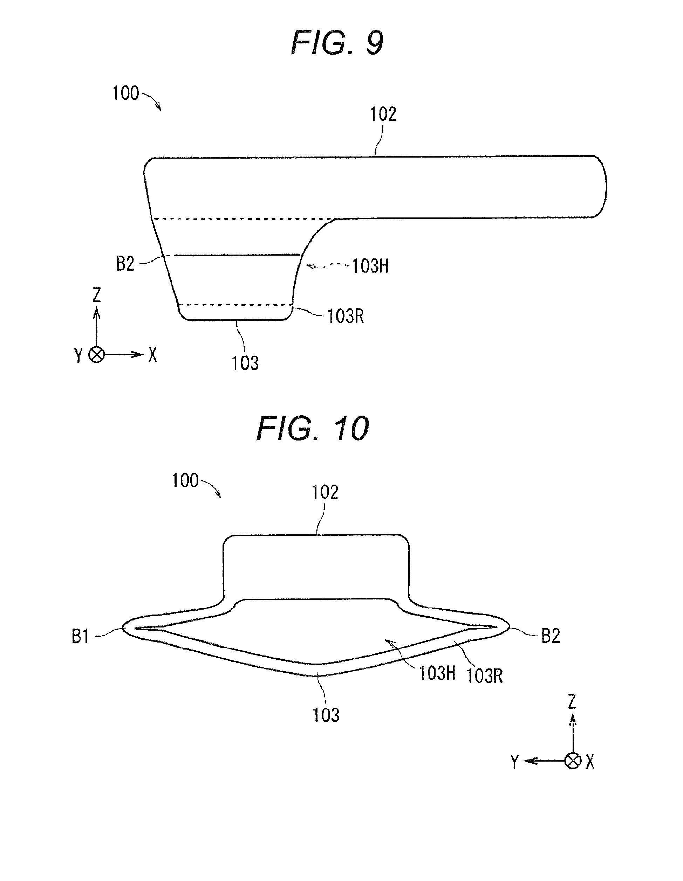

Figs. 9 to 11 are views schematically illustrating an example of a state of attachment of the pulse oximeter 100 to the finger. Figs. 9 and 10 illustrate a mode of the pulse oximeter 100 in a state that a finger is not inserted into the insertion hole 103H. Fig. 11 illustrates a mode of the pulse oximeter 100 in a state that a finger FG1 is inserted into the insertion hole 103H.

For example, when the pulse oximeter 100 is not attached to the finger as illustrated in Figs. 9 and 10, an elastic force, which is generated by the elastic body of the attachment portion 103 for holding the finger inserted into the insertion hole 103H, elastically deforms the annular portion 103R in the Z direction corresponding to the closing direction of the insertion hole 103H. Under this condition, the annular portion 103R may come into a state folded at portions B1 and B2 on the ± Y side as illustrated in Fig. 10, for example.

On the other hand, at the time of attachment of the pulse oximeter 100 to the finger, the annular portion 103R is elastically deformed in such a direction as to expand the insertion hole 103H in the Z direction, by manual operation of a user (health care provider or testee) with resistance to the elastic force generated by the elastic body of the attachment portion 103, so that the finger FG1 can be inserted into the insertion hole 103H in the -X direction as illustrated in Fig. 11. Accordingly, under the condition of attachment of the pulse oximeter 100 to the finger FG1, the finger FG1 is held by the attachment portion 103 via the elastic force generated from the elastic body of the attachment portion 103 in such a direction as to deform the annular portion 103R in the Z direction corresponding to the closing direction of the insertion hole 103H. In this case, the finger FG1 is only required to be inserted into the insertion hole 103H in such a position that the respective lights Lr and Lir can be applied from the light emitting unit 104 to the finger FG1 inserted into the insertion hole 103H, i.e., toward an area between a nail N1 and a distal interphalangeal joint (referred to as first joint as well) J1 of the finger FG1, for example.

### <(1-3) Communication Conversion Device 200>

The communication conversion device 200 is used in combination with the pulse oximeter 100 as a device for converting a communication profile of biological information data. More specifically, the communication conversion device 200 is a communication relay device which receives biological information data obtained by the pulse oximeter 100 in the first communication system, and transmits the biological information data to the display device 300 in the second communication system fitted to the display device 300.

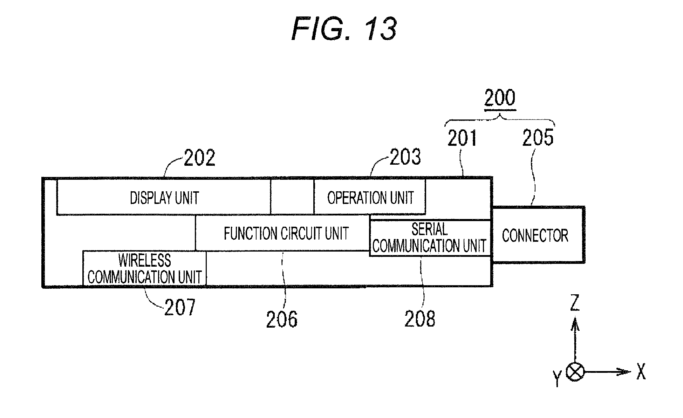

Fig. 12 is a top view illustrating an external appearance of the communication conversion device 200. Fig. 13 illustrates an X-Z cross section of the communication conversion device 200 taken along a dotted line XII-XII in Fig. 12.

As illustrated in Figs. 12 and 13, the communication conversion device 200 includes a body 201 and a connector 205.

The body 201 includes a housing 201h, and further includes a display unit 202, an operation unit 203, a function circuit unit 206, a wireless communication unit 207, and a serial communication unit 208, all units 202 to 207 of which are housed in the housing 201h. The housing 201h has a substantially rectangular solid shape, for example.

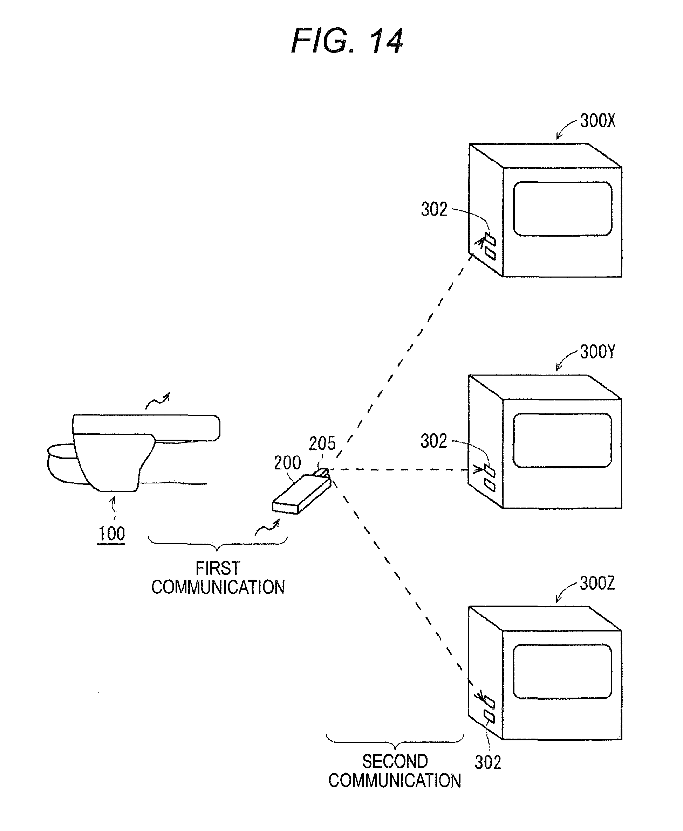

Fig. 14 is a conceptual view illustrating a configuration of the pulse oximetry system 1 including a plurality of the display devices 300 (300X, 300Y, 300Z) having different communication systems (such as communication profiles). Each of the plurality of display devices 300 (300X, 300Y, 300Z) includes a port 302 constituted by a general-purpose port and corresponding to the connector 205. The display unit 202 illustrated in Fig. 12 displays identification information 204 (204X, 204Y, 204Z) in correspondence with the display devices 300 (300X, 300Y, 300Z) illustrated in Fig. 14.

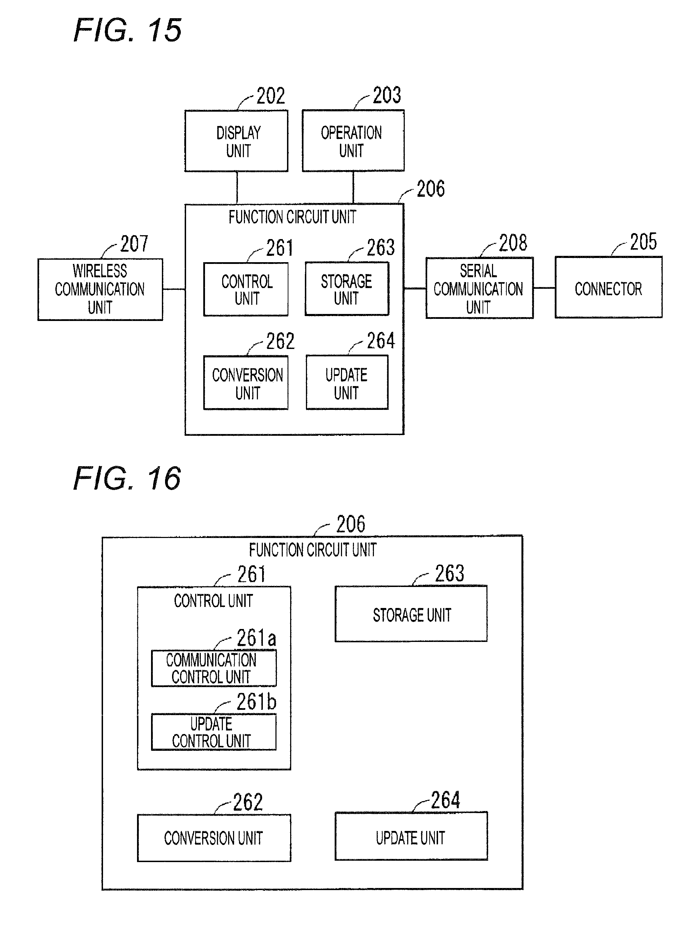

Figs. 15 and 16 are block diagrams illustrating a functional configuration of the communication conversion device 200. Structures of respective parts included in the communication conversion device 200 are hereinafter described in detail with reference to Figs. 12 to 16.

The connector 205 is a physical I/F in the form of USB (Universal Serial Bus) provided on an external part of the communication conversion device 200. Accordingly, when the connector 205 is inserted into the port 302 of any one of the display devices 300 (such as display device 300X), the corresponding display device 300 (such as display device 300X) is electrically connected with the communication conversion device 200. The second communication system according to this embodiment is fitted to serial communication.

A power source supply line is contained in a USB bus connected with the connector 205 according to this embodiment. Accordingly, power is supplied from the display device 300 (such as display device 300X) to the communication conversion device 200 by so-called USB bus power system while the communication conversion device 200 is connected with the display device 300. Accordingly, the necessity of providing a power source unit on the conversion device 200 is eliminated, wherefore the size of the communication conversion device 200 can decrease.

When the communication conversion device 200 is a portable type as in this embodiment, the user is allowed to convey the communication conversion device 200 for use in combination with any one of a plurality of the display devices 300 (data processing devices).

The display unit 202 is configured to display the identification information 204 for each of a plurality of communication system candidates (hereinafter referred to as "candidate communication systems") allowed to be set by the communication conversion device 200 as the second communication system. The identification information 204 (204X, 204Y, 204Z) may be any types capable of supplying information associated with the corresponding candidate communication system to the user. For example, as illustrated in Fig. 12, the identification information 204 in a mode may be information displaying a manufacturer name and a product name of the corresponding display device 300 (300X, 300Y, or 300Z).

The operation unit 203 is configured to receive operation input from the user for selecting the second communication system from the candidate communication systems separately displayed on the display unit 202 for each of the identification information 204. For example, the operation unit 203 in a mode may include a selection button 231 and a decision button 232. In this case, the user selects, via the selection button 231, the identification information 204 (such as identification information 204X) corresponding to one of the display devices 300 (such as display device 300X) from the identification information 204 (204X, 204Y, 204Z) displayed on the display unit 202, and presses the decision button 232 to set a communication system fitted to the display device 300 (such as display device 300X) as the second communication system.

The function circuit unit 206 includes a control unit 261, a conversion unit 262, a storage unit 263, and an update unit 264. The function circuit unit 206 may be any types constituted by various types of electronic parts, integrated circuit parts, a CPU and others. As illustrated in Fig. 16, the control unit 261 includes a communication control unit 261a and an update control unit 261b.

The communication control unit 261a controls the wireless communication unit 207 and the serial communication 208 (described later) while relaying biological information data communication between the respective units 207 and 208. The update control unit 261b controls update of the storage unit 263 achieved by the update unit 264 described later. Each of the communication control unit 261a and the update control unit 261b may be constituted by a dedicated electronic circuit, or realized by a program executed by a microprocessor or a DSP (Digital Signal Processor), for example.

The conversion unit 262 converts data format of the communication profile of the communication system (first communication system) at the time of reception of biological information data from the pulse oximeter 100 into data format fitted to the communication profile of the communication system selected by the user through the operation unit 203. This structure allows conversion of the communication profile of the biological information data from the first communication system to the second communication system, in accordance with operation by the user through the operation unit 203 to select the communication system (second communication system) fitted to the display device 300 (such as display device 300X) corresponding to a transmission destination.

The storage unit 263 is a section for storing various types of information associated with candidate communication systems (such as communication profiles of candidate communication systems, and identification information 204 on candidate communication systems), and is constituted by a readable and writable memory such as a RAM. The display unit 202 displays identification information 204 on the candidate communication systems contained in these sets of information stored in the storage unit 263. Based on the identification information 204, the conversion unit 262 converts data format of biological information data into data format fitted to the communication profile of the communication system selected by the user through the operation unit 203. A communication parameter of the serial communication unit 208 is also set to a parameter fitted to the communication profile of the selected communication system.

As described above, the communication profile of the biological information data after conversion by the communication conversion device 200 (communication profile of second communication system) is selectively set by activation of the display unit 202, the operation unit 203, the conversion unit 262, the storage unit 263, and other various functions. Accordingly, these constituent elements associated with setting of the communication profile (display 202, operation unit 203, conversion unit 262, and storage unit 263) correspond to a "setting unit for setting the communication profile of the second communication system" according to the present invention.

The update unit 264 is connected with a computer containing associated update software to perform a function of updating software provided within the communication conversion device 200. For example, this updating function in a mode may be a function for executing a process for rewriting data and programs stored in the storage unit 263. Updating at least a part of each communication profile of the plurality of candidate communication systems is also included in this updating function. Accordingly, addition and deletion of candidate communication systems, changes of identification information 204, version upgrade of communication profiles and the like are achieved by activation of the function of the update unit 264.

Accordingly, a communication profile fitted to the display device 300 (data processing device) can be added to the storage unit 263 by activation of the function of the update unit 264 at the time of absence of a communication profile fitted to the display device 300 (data processing device) in the communication profiles of the plurality of candidate communication systems stored in the storage unit 263, or at the time of version upgrade of communication profiles. Accordingly, even in case of a data processing device and a pulse oximeter not communicating with each other before updating of the communication conversion device, these data processing device and pulse oximeter can become communicative with each other via the communication conversion device after upgrade.

In addition, usability of the identification information 204 by the user can increase after a change (update) of the identification information 204.

This updating work is achieved by insertion of the connector 205 of the communication conversion device 200 into a general-purpose port (USB port) of a personal computer incorporating updating software corresponding to the update unit 264.

The wireless communication unit 207 is a communication unit which receives biological information data from the wireless communication unit 207 of the pulse oximeter 100 in the first communication system. In this case, the first communication system is fitted to wireless communication, wherefore the necessity of providing a cable or the like (wired connection) for electrically connecting the pulse oximeter 100 and the communication conversion device 200 is eliminated. Moreover, the wireless communication unit 207 is a communication unit corresponding to the communication system fitted to the pulse oximeter 100 (first communication system), wherefore errors caused by the communication system difference are prevented in the first communication.

The serial communication unit 208 is a communication unit which transmits biological information data to the display device 300 (such as display device 300X) in serial format in the communication system selected by the user through the operation unit 203. In this case, an error (incapability of appropriate communication) may be caused by the communication system difference in the second communication when a communication system different from the second communication system (communication system fitted to display device 300X) is accidentally selected at the time of selection of a communication system by the user through the operation unit 203. For avoiding this situation, a notification unit for notifying the user about this error may be provided on the communication conversion device 200. Means for issuing this notification may be a circuit for displaying an error on the display unit 202, or a circuit for blinking an additionally provided light emitter such as an LED, for example. The serial communication unit 208 corresponds to "transmitting means" according to the present invention.

### <(1-4) Display Device 300>

The display device 300 is a device which receives biological information data from the communication conversion device 200, and displays medical information on the display unit 301 based on the biological information data. The display device 300 may be constituted by various types of known display devices.

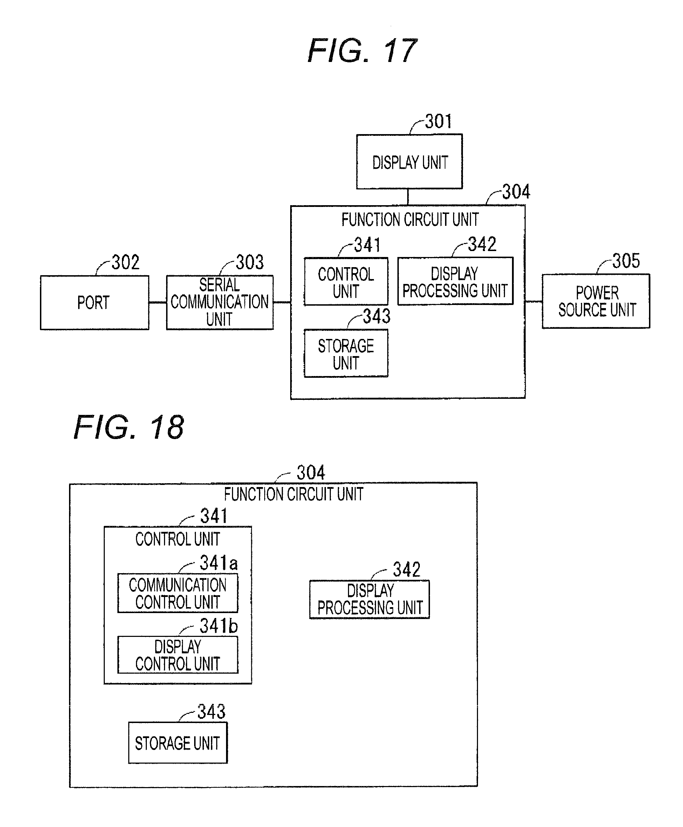

Figs. 17 and 18 are block diagrams illustrating a functional configuration of the display device 300.

As illustrated in Figs. 1, 14, 17, and 18, the display device 300 includes the display unit 301, a port 302, a serial communication unit 303, a function circuit unit 304, and a power source unit 305.

The display unit 301 displays medical information on the display unit 301 based on biological information data on the testee obtained by the pulse oximeter 100.

The port 302 is a general-purpose port (such as USB port) provided on an external part of the display device 300, and used at the time of serial communication with the communication conversion device 200. More specifically, the communication conversion device 200 and the display device 300 are electrically connected and brought into a communicative state by insertion of the connector 205 into the port 302.

The serial communication unit 303 communicates with the serial communication unit 208 for biological information data communication in the second communication system. The second communication system is fitted to serial communication. The second communication is realized by the serial communication unit 208 and the serial communication unit 303. The second communication system may be a system peculiar to the model or the manufacturer of the display device 300.

The function circuit unit 304 includes a control unit 341, a display processing unit 342, and a storage unit 343. The electronic circuit 304 may be any types of circuit including various electronic parts, integrated circuit parts, a CPU and others. As illustrated in Fig. 18, the control unit 341 includes a communication control unit 341a and a display control unit 341b.

The communication control unit 341a controls communication between the serial communication unit 208 and the serial communication unit 208 (second communication). The display control unit 341b controls display of biological information data received by the display device 300 and displayed on the display unit 301. Each of the communication control unit 341a and the display control unit 341b may be constituted by a dedicated electronic circuit, or may be realized by a program executed by a microprocessor or a DSP (Digital Signal Processor), for example.

The display processing unit 342 performs predetermined data processing for biological information data received from the communication conversion device 200 to convert data format of the biological information data into data format appropriate for display on the display unit 301.

The storage unit 343 which stores biological information data transmitted to the display device 300 is constituted by a readable and writable memory such as a RAM. Accordingly, when a process such as an analysis process is performed for biological information data stored in the storage unit 343 by using an external device outside the pulse oximetry system 1, for example, the corresponding biological information data stored in the storage unit 343 may be transmitted to this external device.

The power source unit 305 may be any types capable of supplying operation power to respective circuits of the display device 300, wherefore various known configurations are adoptable for the power source unit 305. For example, the power source unit 305 may have a configuration containing a general-purpose plug connectable with a power outlet.

### <(1-5) Summary of This Embodiment>

Fig. 19 is a block diagram illustrating a functional configuration of the pulse oximetry system 1 according to this embodiment (see Figs. 7, 15, and 17). Discussed hereinbelow with reference to Fig. 19 is a summary of the pulse oximetry system 1 according to this embodiment.

The first communication system is used for communication between the pulse oximeter 100 and the communication conversion device 200 (first communication). On the other hand, the second communication system is used for communication between the communication conversion device 200 and the display device 300 (second communication). According to this embodiment, the first communication system is fitted to wireless communication, while the second communication system is fitted to serial wired communication.

The communication conversion device 200 includes constituent elements for converting a communication profile (display unit 202, operation unit 203, conversion unit 262, storage unit 263 and others). Accordingly, when the communication profile of the first communication system is different from the communication profile of the second communication system, conversion (relay) of the communication profile of biological information data is allowed from the first communication system to the second communication system by the communication conversion device 200. This structure realizes real-time communication via the communication conversion device 200 even when the communication profiles of the pulse oximeter 100 and the display device 300 are different.

Under a generally communicative state of the pulse oximetry system 1 thus achieved, biological information data on a test living body is created based on measurement conducted by using the pulse oximeter 100. The signal processing unit 62 further executes various signal processes for the created biological information data. The pulse oximeter 100 transmits the resultant biological information data to the communication conversion device 200 in real time in the first communication system.

The communication conversion device 200 having received the biological information data from the pulse oximeter 100 in the first communication system converts the communication profile of the received biological information data into a communication profile of a communication system (second communication system) fitted to the display device 300 corresponding to a transmission destination of the biological information data. More specifically, the user selects the identification information 204 corresponding to the second communication system through the operation unit 203 from respective sets of the identification information 204 on candidate communication systems displayed on the display unit 202, and converts the communication profile of the biological information data into the communication profile of the second communication system.

The biological information data converted into data of the communication profile of the second communication system is transmitted from the communication conversion device 200 to the display device 300 via the connector 205 and the port 302. As a result, medical information is displayed on the display device 300 based on the biological information data.

As described above, biological information data is transmitted from the pulse oximeter 100 to the display device 300 via the communication conversion device 200 according to the pulse oximetry system 1 in this embodiment. The communication profile of the biological information data is converted through the communication conversion device 200 in accordance with selection of a communication system by the user through the operation unit 203 from candidate communication systems stored in the storage unit 263.

Accordingly, the following two advantages are offered according to the pulse oximetry system 1 in this embodiment.
1) In case of presence of a communication profile fitted to the display device 300 in communication profiles of a plurality of candidate communication systems stored in the storage unit 263, communication is realized via the communication device 200 even when the communication systems of the display device 300 and the pulse oximeter 100 are different.
2) In case of absence of a communication profile fitted to the display device 300 (such as display device 300) in the communication profiles of the plurality of candidate communication systems stored in the storage unit 263, a communication profile fitted to the display device 300 may be added to the candidate communication systems by the function of the update unit 264. This updating work also produces a state of communication between the display device 300 and the pulse oximeter 100 via the communication conversion device 200.

### <(2) Modified Examples>

The present invention is not limited to the embodiment described herein. Various modifications, improvements and others may be made without departing from the scope of the subject matters of the present invention.

According to this embodiment, the pulse oximetry system 1 is constituted by the pulse oximeter 100, the communication conversion device 200, and the display device 300. However, the present invention is not limited to this configuration. For example, the pulse oximetry system 1 may include, instead of the display device 300, a medical operation device (such as breathing management device and artificial heart-lung machine) which performs predetermined medical operation for a test living body based on biological information data.

More specifically, in constituting the pulse oximetry system 1, such a subsystem (module) 2 (see Fig. 19) is generally adopted which includes, as chief elements, the pulse oximeter 100 for creating biological information data, and the communication conversion device 200 converting the communication profile of the biological information data into a profile fitted to a data processing device corresponding to a transmission destination. The data processing device receiving the biological information data from the subsystem 2 may be various known types of data processing devices such as the display device 300 and a medical operation device.

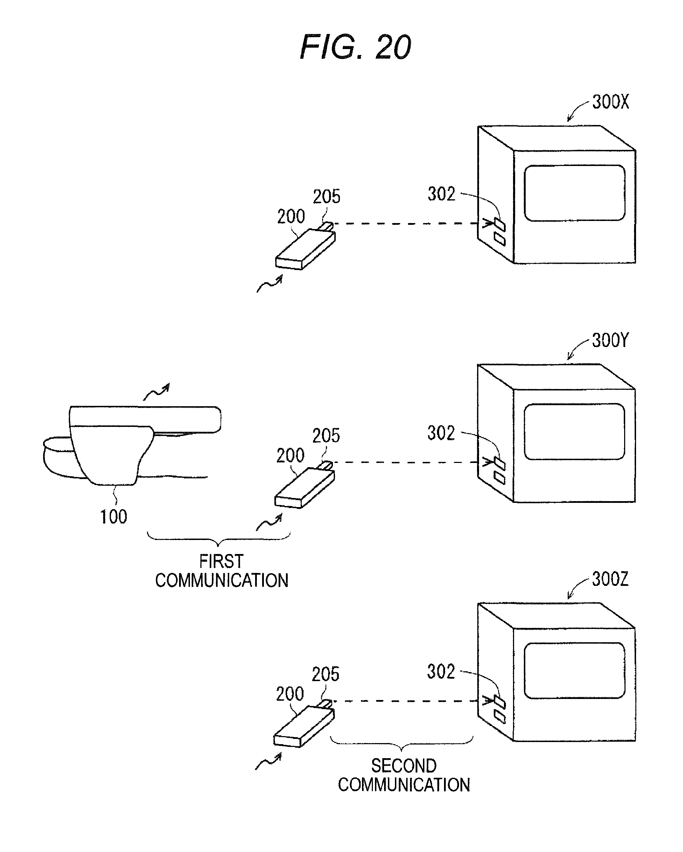

According to this embodiment, the pulse oximetry system 1 is so configured that biological information data created by the pulse oximeter 100 is transmitted to the one display device 300 (data processing device). However, the present invention is not limited to this configuration. As illustrated in Fig. 20, biological information data may be simultaneously transmitted to the plurality of the display devices 300 (300X, 300Y, 300Z) using a plurality of the communication conversion devices 200 (200X, 200Y, 200Z). In this case, the communication profile of the second communication system may be individually set for each of the communication conversion devise 200 (200X, 200Y, 200Z), and therefore may be a different communication profile for each of the display devices 300 (300X, 300Y, 300Z).

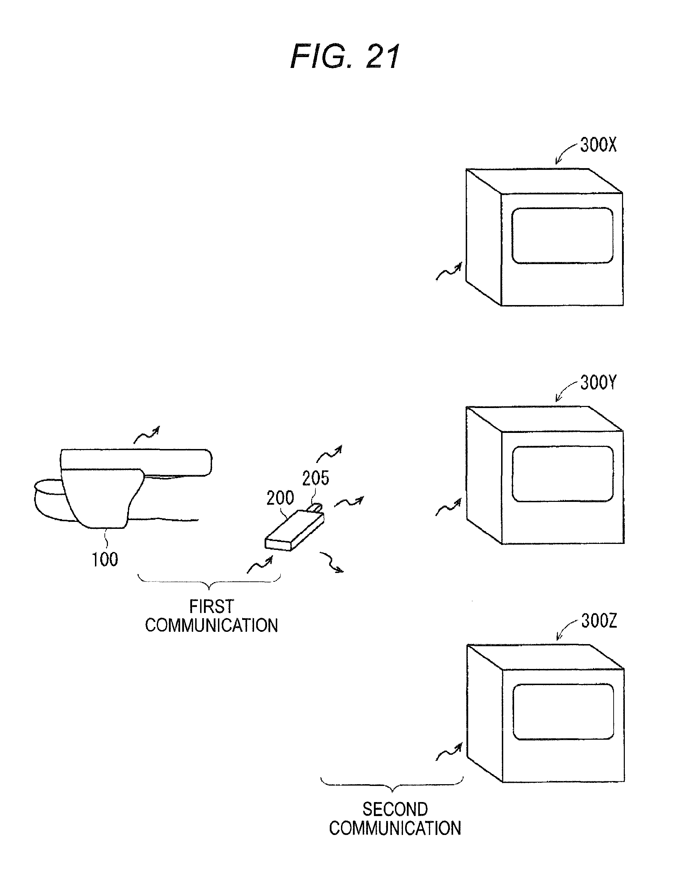

According to this embodiment, the second communication is achieved by insertion of the connector 205 into the port 302. However, the present invention is not limited to this configuration. As illustrated in Fig. 21, the second communication may be realized by a general-purpose wireless communication system (such as "Bluetooth"). In this case, the communication conversion device 200 may be used in combination with various types of data processing devices capable of realizing wireless communication in the corresponding general-purpose wireless communication system.

According to this structure, biological information data is simultaneously transmittable from the one communication conversion device 200 to the plurality of display devices 300 (300X, 300Y, 300Z) having the second communication system fitted to the corresponding general-purpose wireless communication system. However, when only one type of the communication profile is used for simultaneous transmission of the biological information data from the communication conversion device 200, the plurality of display devices 300 (300X, 300Y, 300Z) need to have the same communication profile. For overcoming this problem, the communication conversion device 200 may be configured to handle a plurality of communication profiles to allow parallel transmission. In this case, biological information data is transmittable to a plurality of medical devices of various types having different communication profiles for transmission in parallel.

According to the pulse oximetry system 1 in the foregoing one embodiment, the pulse oximeter 100 includes the signal processing unit 62 (I/V conversion unit 162a, A/D conversion unit 162b, and analysis processing unit 162c). However, the present invention is not limited to this configuration. A part or all of the configuration of the signal processing unit 62 may be provided not on the pulse oximeter 100, but on the communication conversion device 200 or the display device 300. In this case, reduction of the size of the pulse oximeter 100, and thus reduction of a burden on the testee caused by attachment (minimal invasion) are achievable when the analysis processing unit 162c is disposed on the communication conversion device 200, for example.

According to this embodiment, the first communication system is fitted to wireless communication. However, the present invention is not limited to this configuration. Communication between the pulse oximeter 100 and the communication conversion device 200 may be realized by wired communication using a flexible cable or the like. It is preferable, however, that wireless communication is adopted as in this embodiment in view of the high degree of freedom in motion of the body of the testee.

According to this embodiment, the second communication system is fitted to serial communication. However, the present invention is not limited to this configuration. Communication between the communication conversion device 200 and the display device 300 (data processing device) may be realized by parallel communication. However, serial communication is more preferable in the point that "no problem of clock deviation occurs between transmission routes", or that "advantages of space saving and avoidance of noise entrance from the surroundings are easily offered by reduction of the number of cables", for example.

According to this embodiment, transmission from the pulse oximeter 100 to the communication conversion device 200 is chiefly discussed as the first communication. In more detail, however, the first communication adopted herein includes transmission from the communication conversion device 200 to the pulse oximeter 100 as well (configuration for bidirectional communication). In this case, the pulse oximeter 100 and the communication conversion device 200 are paired by bidirectional communication, in which condition connection of the first communication is automatically, or semiautomatically established after the pairing.

The test living body may be an animal instead of a human. In this case, the pulse oximeter 100 is modified into a shape appropriate for a finger of the animal. The communication conversion device 200 is available in common in either case of a human or an animal corresponding to a test living body.

Needless to say, all or a part of configurations of the foregoing embodiment and various types of modified examples may be combined in appropriate manners within a range not producing inconsistency.

### Reference Signs List

- 1: pulse oximetry system
- 2: subsystem
- 102: body
- 103: attachment portion
- 104: light emitting unit
- 105: light receiving unit
- 106: function circuit unit
- 109: wireless communication unit
- 162: signal processing unit
- 200: communication conversion device
- 201: body
- 202: display unit
- 203: operation unit
- 204: identification information
- 205: connector
- 206: function circuit unit
- 207: wireless communication unit
- 208: serial communication unit
- 300: display device
- 301: display unit
- 302: port
- 303: serial communication unit
- 304: function circuit unit

## Claims

1. A pulse oximetry system comprising:
(a) a pulse oximeter attached to a finger of a test living body to create biological information data containing information for specifying oxygen saturation in blood of the test living body, and transmit the biological information data in a predetermined first communication system;
(b) a data processing device that performs predetermined data processing for the biological information data; and
(c) a communication conversion device that includes a storage unit storing each communication profile of a plurality of candidate communication systems, and a setting unit selecting a second communication system fitted to communication with the data processing device from the plurality of candidate communication systems, and setting a communication profile of the second communication system, wherein the communication conversion device transmits the biological information data to the data processing device in the second communication system after receiving the biological information data in the first communication system from the pulse oximeter.

2. The pulse oximetry system according to claim 1, wherein the communication conversion device is portable.

3. The pulse oximetry system according to claim 1 or 2, wherein
the communication conversion device further includes an update unit for updating software provided within the communication conversion device, and
at least a part of the communication profiles of the plurality of candidate communication systems stored in the storage unit is updatable by the update unit.

4. The pulse oximetry system according to any one of claims 1 to 3, wherein the communication conversion device further includes
a display unit that displays identification information on the plurality of candidate communication systems stored in the storage unit, and
an operation unit that receives operation input for selecting the second communication system from the plurality of candidate communication systems separately displayed on the display device for each of the identification information.

5. The pulse oximetry system according to any one of claims 1 to 4, wherein
wireless communication is used for communication of the biological information data between the pulse oximeter and the communication conversion device, and
the first communication system is fitted to wireless communication.

6. The pulse oximetry system according to any one of claims 1 to 5, wherein
serial communication is used for communication of the biological information data between the communication conversion device and the data processing device, and
each of the plurality of candidate communication systems is fitted to serial communication.

7. The pulse oximetry system according to any one of claims 1 to 6, wherein
communication between the pulse oximeter and the data processing device via the communication conversion device is realized in a state of electrical connection between a connector provided on an external part of the communication conversion device and a port provided on an external part of the data processing device, and
the port is a general-purpose port provided on each of a plurality of devices available as the data processing device in common.

8. The pulse oximetry system according to any one of claims 1 to 6, wherein
a general-purpose wireless communication system is used for communication of the biological information data between the communication conversion device and the data processing device, and
the second communication system is fitted to the general-purpose wireless communication system.

9. The pulse oximetry system according to any one of claims 1 to 8, wherein the data processing device is a display device that displays medical information obtained based on the biological information data.

10. The pulse oximetry system according to any one of claims 1 to 8, wherein the data processing device is a medical operation device that performs predetermined medical operation for the test living body based on the biological information data.

11. A subsystem for constructing a pulse oximetry system that includes
(a) a pulse oximeter attached to a finger of a test living body to create biological information data containing information for specifying oxygen saturation in blood of the test living body, and transmit the biological information data in a predetermined first communication system, and
(b) a communication conversion device that includes a storage unit storing each communication profile of a plurality of candidate communication systems, and a setting unit selecting a second communication system fitted to communication with a data processing device corresponding to a data transmission destination from the plurality of candidate communication systems, and setting a communication profile of the second communication system, wherein the communication conversion device transmits the biological information data to the data processing device in the second communication system after receiving the biological information data in the first communication system from the pulse oximeter.

12. A communication conversion device used in combination with a pulse oximeter attached to a finger of a test living body to create biological information data containing information for specifying oxygen saturation in blood of the test living body, and transmit the biological information data in a predetermined first communication system, the communication conversion device comprising:
a storage unit storing each communication profile of a plurality of candidate communication systems;
a setting unit selecting a second communication system fitted to communication with a data processing device corresponding to a transmission destination of the biological information data from the plurality of candidate communication systems, and setting a communication profile of the second communication system; and
transmitting means that transmits the biological information data to the data processing device in the second communication system after receiving the biological information data in the first communication system from the pulse oximeter.
